# EUROPÄISCHE PATENTSCHRIFT

(11) **EP 1 381 387 B1**
(45) Veröffentlichungstag und Bekanntmachung des Hinweises auf die Patenterteilung: **08.08.2007**
(21) Anmeldenummer: 02740502.6
(22) Anmeldetag: 24.04.2002
(51) Int. Cl.: A61K 39/00, A61K 35/14, A61P 35/00

(54) **VERWENDUNG VON STIMULIERTEN MONONUKLEAREN ZELLEN DES PERIPHEREN BLUTES ZUR BEHANDLUNG VON KREBSERKRANKUNGEN**
USE OF STIMULATED PERIPHERAL BLOOD MONONUCLEAR CELLS FOR TREATING CANCEROUS DISEASES
UTILISATION DE CELLULES MONONUCLEAIRES DU SANG PERIPHERIQUE STIMULEES POUR TRAITER DES AFFECTIONS CANCEREUSES

(30) Priorität: 26.04.2001 DE 10120505
(43) Veröffentlichungstag der Anmeldung: 21.01.2004
(73) Patentinhaber: Wank, Rudolf, 80469 München (DE)
(72) Erfinder: Wank, Rudolf, 80469 München (DE)
(74) Vertreter: Wibbelmann, Jobst
(86) Internationale Anmeldenummer: PCT/EP2002/004524
(87) Internationale Veröffentlichungsnummer: WO 2002/087612

(56) Entgegenhaltungen:
- WO-A-95/20349
- WO-A-96/29394
- WO-A-99/50393
- TAKAYAMA TADATOSHI ET AL: "Adoptive immunotherapy to lower postsurgical recurrence rates of hepatocellular carcinoma: A randomised trial." LANCET (NORTH AMERICAN EDITION), Bd. 356, Nr. 9232, 2000, Seiten 802-807, XP002215496 ISSN: 0099-5355 in der Anmeldung erwähnt

## Beschreibung

Die Erfindung betrifft die Verwendung von über eine "Kaskaden-Gedächtnisprägung" stimulierten oder aktivierten mononukleären Zellen des peripheren Blutes zur Bereitstellung eines Mittels zur Behandlung von Krebserkrankungen.

Im Rahmen der Immuntherapie zur Bekämpfung von Krebserkrankungen stellt die Stimulierung von naiven (nicht stimulierten) T-Lymphozyten einen wichtigen Ansatz dar. Eine optimale Aktivierung von naiven T-Zellen erfordert eine spezifische antigene Stimulierung des αβ-T-Zellrezeptors (TCR) durch den MHC-Peptid/Antigen-Komplex (MHC = großer Histokompatibilitätskomplex) in Verbindung mit costimulatorischen Signalen. Die Abwesenheit der Costimulierung führt zu einer funktionellen Deaktivierung der T-Zellen. Die costimulatorischen Signale werden am besten von den Antigen-präsentierenden Zellen (APC) bereitgestellt.

Die APC sind mit einer Reihe von costimulatorischen Molekülen ausgestattet, die substantiell zur Verstärkung der spezifischen Aktivierung von T-Zellen über den TCR beitragen. Aktivierungs-induzierende komplementäre Moleküle konnten auf T-Lymphozyten und APC in Form von Membranproteinen identifiziert werden. Auf den T-Lymphozyten werden die Membranproteine unterteilt in T-Zell-Corezeptoren, wie das CD4-Molekül oder das CD8-Molekül, und in interzelluläre Adhäsionsmoleküle-1,2,3 (I-CAM-1,2,3). Letztere werden von APC und T-Zellen exprimiert und treten mit der Leukozytenfunktion Antigen-1 in Wechselwirkung (LFA-1-Rezeptorfamilie CD11a/CD18), die ebenfalls von APC und T-Zellen exprimiert wird. Die CD2-Moleküle, die von den meisten T-Zellen exprimiert werden, reagieren mit CD58 (LFA-3). Die T-Zellen wie auch die APC exprimieren die Adhäsionsmoleküle LFA-1 und ICAM-1, die für den intensiven Kontakt zwischen APC und T-Zellen von größter Bedeutung sind. Kürzlich wurden weitere Moleküle mit costimulatorischer Funktion identifiziert, wie z.B. CD27/CD27L (expandieren T-Zellen nach CD28 Costimulation), SLAM (Cdw150, Mitglied der CD2-Familie, erhöht die Interferon γ (IFN γ)-Produktion und Proliferation der Gedächtnis-T-Zellen) und OX40 (CD134, fördert die Helfer-2-T-Zellen-(T_{H}2)-Antwort und unterstützt die T_{H}1- und T_{H}2-Proliferation). Die genaue Funktion weiterer costimulatorischer Moleküle wird weiterhin diskutiert, kein Zweifel besteht jedoch an der Bedeutung der Aufklärung für das Verständnis der individuellen Immunantwort (J.E.M. van Leuwen, L. Samelson, T cell antigen-receptor transduction, Curr. Opin. Immunol. 11:242-248 (1999)).

Krebszellen liefern nur in geringem Maße costimulatorische Signale. Des weiteren neigen Krebszellen dazu, die Expression von MHC-Molekülen drastisch einzuschränken und so die Erkennung der Tumorpeptide im MHC-Kontext zu verhindern.

Mehrere Strategien wurden entwickelt, um der geringen Stimulierung der Krebszellen entgegenzuwirken und die naiven (nicht stimulierten) T-Lymphozyten gegen die relevanten Tumorantigene zu prägen. Viele Bemühungen gingen in Richtung einer Verbesserung der costimulatorischen Signale mit Hilfe der Gentechnik, z.B. durch Transfektion von Tumorzellen mit T-Zellen aktivierenden Genen. Des weiteren sind viele Anstrengungen auf die Isolierung und Identifizierung von Tumorpeptiden gerichtet. Solche Tumorpeptide werden zur Beladung von APC verwendet, um eine effiziente Präsentation der MHC-Tumorpeptid-Komplexe gegenüber den T-Zellen zu ermöglichen. Dadurch soll eine Aktivierung der T-Zellen und die Induktion von T-Zellen-Gedächtnis erreicht werden. Mit Antigen beladene APC werden bereits in klinischen Forschungszentren in Patienten reinfundiert, um naive T-Zellen anzuziehen und gegen das Tumorantigen zu prägen. Bis jetzt wurde die intensive Beladung der APC entweder mit Tumorpeptiden/Antigenen oder über eine Inkubation mit Krebszellen vorgenommen.

Der sehr geringe Erfolg dieser therapeutischen Strategien wird durch vier Hauptprobleme, die einzeln oder in Kombination auftreten können, bedingt: 1) die Schwierigkeit, ein Tumorantigen zu identifizieren, welches in jedem Individuum eine starke Immunantwort induziert, 2) der Verlust von ausgewählten Präsentations-Antigenen auf den Tumorzellen, der durch den Verlust der Präsentation einzelner MHC-Moleküle bewirkt wird, welche diese Tumorantigene/Peptide präsentierten, 3) die Fähigkeit von Krebszellen, naive Zellen durch Zytokine zu inaktivieren und 4) das nicht ausreichende Auftreten von costimulatorischen Signalen.

Eine andere Strategie zur Behandlung von u.a. Krebserkrankungen wird in der WO95/20649 beschrieben. Darin wird ein Verfahren zur Herstellung von in vitro generierten Immunzellen offenbart, bei dem mononukleäre Zellen des peripheren Blutes (PBMC) mit einem löslichen CD3-Antikörper inkubiert werden, und der Überstand zur Inkubation von naiven PBMC verwendet wird. Die so erhaltenen immunreaktiven Zellen, deren T-Zellpopulation vorwiegend aus Helfer-T-Zellen (CD4⁺) und zytotoxische T-Zellen (CD8⁺) besteht, werden in den Patienten reinfundiert.

Es besteht daher weiterhin ein Bedarf für ein Mittel zur Behandlung von Krebserkrankungen.

Erfindungsgemäß können die über die Kaskaden-Gedächtnisprägung stimulierten PBMC (sogenannte CAPRI-Zellen) zur Bereitstellung eines Mittels zur Behandlung von Krebserkrankungen eingesetzt werden.

Gemäß der Erfindung werden naive T-Zellen im PBMC-Verband eines Patienten in vitro aktiviert, wie in Anspruch 1 definiert, und gegen die eigenen Tumorzellen geprägt; dabei kommen PBMC zum Einsatz, die nicht einer vorherigen Tumorantigen-Beladung unterworfen werden mussten. Für diese in vitro-Prägung besteht somit nicht die Notwendigkeit, Tumorzellen zu verwenden oder die spezifischen Tumorantigene/Peptide zu identifizieren.

Bei der Kaskaden-Gedächtnisprägung werden naive PBMC mittels CD3 Antikörper und Optional anderer T-Lymphozyten stimulierender Agenzien primär stimuliert, und diese primär stimulierten PBMC naiven PBMC zugesetzt, wodurch die naiven PBMC ihrerseits stimuliert werden, und die daraus resultierenden CAPRI-Zellen ("cascade primed" Zellen) erfindungsgemäß zur Bereitstellung eines Mittels zur Behandlung von Krebserkrankungen eingesetzt werden können.

Unter T-Lymphozyten stimulierende Agenzien werden CD3-Antikörper, B7-Antikörper, Lektine, Calciumionophore, allogene Zellen, xenogene Zellen und ähnliches verstanden. Die verschiedenen Agenzien können allein oder in Kombination für die Primärstimulierung eingesetzt werden. Die Primärstimulierung erfolgt über die Verwendung von CD3-Antikörpern, insbesondere von immobilisierten CD3-Antikörpern. Die Stimulierung der naiven PBMC, resp. der T-Lymphozyten im PBMC-Verband, kann aber auch durch die anderen aufgelisteten Maßnahmen ergänzt werden, die in der WO99/50393 des Anmelders, auf die hier ausdrücklich verwiesen wird, ausführlich beschrieben werden. Im folgenden wird die Erfindung am Beispiel der CD3-Antikörper vermittelten Primärstimulierung näher beschrieben, ohne daß die Erfindung darauf beschränkt werden soll.

Die Primärstimulierung der T-Lymphozyten im PBMC-Verband kann mittels CD3-Antikörper unter nachfolgendem Zusatz von Interleukin-2 (IL-2) (sog. IL-2 Unterstützungsphase) erfolgen. Nach einer Inkubationszeit für die Primärstimulierung von 4-8 h werden den stimulierten PBMC (resp. den stimulierten T-Lymphozyten im PBMC-Verband) naive PBMC zugesetzt. Nach einer weiteren Inkubationsdauer von mindestens 18-24 h (sog. Prägungsphase) ist die Stimulierung der naiven PBMC abgeschlossen, so daß die Zellpräparation unter Zusatz von IL-2 ca. 3 Tage expandiert und/oder erfindungsgemäß eingesetzt werden kann (sog. Expansionsphase). Während dieser weiteren Inkubation erfolgt eine kontinuierliche Weiterstimulierung der bereits primär stimulierten Zellen, während die neu hinzugefügten naiven PBMC hauptsächlich über die in dem ersten Schritt primär stimulierten Zellen stimuliert werden. Das zahlenmäßige Verhältnis der primär stimulierten PBMC zu den naiven PBMC beträgt in der Regel etwa 1:1. Das Verfahren zur Herstellung von CAPRI-Zellen und deren Verwendung zur Behandlung von mit dem Gehirn assoziierten Erkrankungen, Störungen und Schädigungen wird bereits in der WO99/50393 des Anmelders angesprochen.

Gemäß einer weiteren Ausführungsform können während der IL2-Unterstützungsphase und/oder während der Prägungsphase andere Zytokine, wie z.B. IL-4 und/oder GMCSF ("IL-4 CAPRI-Zellen") oder Interferone ("IFN γ CAPRI-Zellen") zugegeben werden, um so die Expression anderer Peptide durch die APC zu erreichen und die Expansion anderer APC- oder T-Zellsubpopulationen zu fördern. Die CAPRI-Zellen können in einer neuen Runde der Prägung von naiven Zellen mit anschließender Expansion eingesetzt werden.

Gemäß einer weiteren Ausführungsform kann das Verhältnis der cokultivierten aktivierten PBMC und der naiven PBMC von 1:1 zu 1:10 oder 10:1 geändert werden, d.h. hundertfach.

Gemäß einer weiteren Ausführungsform können auch allogene (fremde) Immunzellen bei der Herstellung von CAPRI-Zellen eingesetzt werden. Bei verwandten Individuen, bei denen ein oder zwei HLA-Haplotypen (HLA = humaner großer Histokompatibilitätskomplex) übereinstimmen, kann eine ausreichende Immunzellenkooperation zwischen den naiven T-Zellen und den aktivierten APC gewährleistet werden. Bei einem unterschiedlichen Haplotyp kann eine zusätzliche Stimulierung auftreten (Allo-Stimulierung). Die Allo-Stimulierung von einem Haplotypenunterschied wurde vor längerer Zeit beschrieben (M.J. Sheehy, P.M. Sondel, M.L. Bach, R. Wank, F.H. Bach, HL-A LD (lymphocyte defined) typing: a rapid assay with primed lymphocytes, Science 188:1308-1310 (1975)). Zellen von nicht verwandten Individuen können ebenfalls verwendet werden, falls zwischen den allogenen Zellen und den Tumorzellen des Patienten genügend HLA-Übereinstimmung für eine HLA-restringierte Lyse besteht. Die folgenden Kombinationen können vorteilhafterweise bei der Behandlung von Krebserkrankungen eingesetzt werden:
1) Aktivierte APC des Patienten + naive PBMC eines Individuums, das die gleichen relevanten HLA-Allele aufweist: Die generierten Gedächtnis-Effektorzellen sind nicht vom Patienten, folglich Allo-CAPRI-Zellen. Dies stellt die häufigste Variante dar, da in den meisten Fällen die APC des Patienten die Tumorpeptide am besten präsentieren.
2) APC von einem "resistenten" Individuum (z.B. resistent gegen das humane Papilloma-Virus, HPV, R. Wank, C. Thomssen, High risk of sqamous cell carcinoma of the servix for women with HLA-DQw3, Nature 352:723-725 (1991)) werden zur Prägung von naiven PBMC einer Patientin mit durch HPV induziertem Gebärmutterhalskrebs verwendet: Die APC sind fremd (Allo), die generierten Gedächtnis-Effektorzellen sind vom Patienten, folglich Allo-APC-CAPRI.
3) Die APC wie auch die generierten Gedächtnis-Effektorzellen sind fremd (Allo), folglich Allo-allo-CAPRI-Zellen. Die Allo-allo-CAPRI-Zellen sind optimal, wenn sowohl der Patient als auch der allogene Donor mit dem gleichen karzinogenen Faktor in Berührung gekommen sind und der allogene Donor dabei eine überlegene Immunantwort aufwies.

Die Kinetik der allogenen oder semi-allogenen CAPRI-Kombinationen unterscheidet sich nicht von der der "normalen" CAPRI-Methode.

Bei einer weiteren bevorzugten Ausführungsform der Erfindung werden CAPRI-Zellen in Kombination mit CD3-aktivierten Zellen oder mit CD3-aktivierten Zellen, die durch IFN γ-Zugabe in Richtung der T_{H}1-Population oder durch Zugabe von IL-4 in Richtung der T_{H}2- und CD8⁺ T-Zell-Populationen polarisiert worden waren, verabreicht.
Dies hat zum Teil historische Gründe, da die nur CD3-aktivierten Zellen vor der Erfindung der CAPRI-Zellen bei anderen Indikationen eingesetzt wurden. Da die Behandlung mit nur CD3-aktivierten Zellen eine vorteilhafte Wirkung auf eine geringe Rate der Tumor-Wiederkehr hat (T. Takayama et al., Adoptive immunotherapy to lower postsurgical recurrence rates of hepatocellular carcinoma: a randomised trial, Lancet 356:802-807 (2000)), möglicherweise aufgrund der erhöhten Zahl an CD8⁺ T-Zellen, können diese Zellen zur Unterstützung der CAPRI-Zellen verabreicht werden. Zudem können CD3-aktivierte Zellen bei Patienten mit depressiven Störungen erfolgreich eingesetzt werden, wie bereits in DE 19814701 des Anmelders beschrieben worden ist. Diese Effekte können auch erfolgreich bei der Bekämpfung der negativen Auswirkungen der Chemotherapie oder der Bestrahlung ausgenutzt werden.

Ohne daß beabsichtigt wird, auf eine bestimmte Theorie zur Erklärung der feststellbaren Wirkung der CAPRI-Zellen zur Bekämpfung von Tumorzellen festgelegt zu werden, wird der im folgenden näher erläuterte Wirkungsmechanismus in Betracht gezogen.

Bei der Stimulierung über die Kaskaden-Gedächtnisprägung wird davon ausgegangen, daß die Induktion der Immunantwort mit einer allgemeinen Aktivierung der naiven T-Zellen im PBMC-Verband (mittels CD3-Antikörper) gestartet wird. Diese aktivierten T-Zellen sekretieren eine Reihe von Zytokinen, welche die APC (Monozyten/Makrophagen, dendritische Zellen, B-Lymphozyten) aktivieren. Entsprechend sekretieren aktivierte APC eine Vielzahl von Zytokinen und exprimieren vermehrt costimulatorische Liganden. Noch bedeutsamer aber ist die effizientere Präsentation von Peptiden endogenen oder exogenen Ursprungs durch die APC. Bei solchen Peptiden kann es sich um Tumorpeptide handeln, die von Krebszellen generiert werden, und die nun vermehrt von den APC präsentiert werden. Es wird davon ausgegangen, daß die Stimulierung der anschließend zugesetzten naiven Zellen ganz überwiegend durch die aktivierten APC der primär stimulierten PBMC erfolgt, d.h. hochspezifisch über den αβ-TCR der T-Lymphozyten (CD4⁺ Helferzellen und CD8^{*} Killerzellen) in der naiven PBMC-Population.

Im folgenden werden die verschiedenen Phasen, die während der Kaskadengedächtnis-Prägung durchlaufen werden, noch näher beleuchtet.
1) T-Zellaktivierung via CD3 - Induktion der Zytokinsekretion Die Bindung von T-Lymphozyten an immobilisierte CD3-Antikörper und die Zugabe von IL-2 führen zu einer Aktivierung der T-Lymphozyten über die invarianten CD3-Polypeptide, die mit dem αβ-TCR assoziiert sind. Die Aktivierung induziert auch eine Sekretion von Zytokinen durch die T-Zellen. In dieser Phase kann der Typus der T-Zellenpopulation durch Addition von Zytokinen beeinflußt und expandiert werden, so kann z.B. durch Zugabe von IL-4 eine spätere Amplifizierung in Richtung der T_{H}2-Zellen gelenkt werden. In dieser Phase kann auch eine signifikante Vermehrung der CD8⁺ T-Zellen beobachten werden.
2) Aktivierung der APC
   Die Zytokine der T-Zellen aktivieren die APC, Monozyten differenzieren zu Makrophagen und dendritische Zellen reifen. Die Zytokine, die von den unterschiedlich aktivierten T-Zellen produziert werden, beeinflussen den vorherrschenden Typus der APC und beeinflussen auch die Art der enzymatischen Untereinheiten, die in den APC aktiviert werden. Entsprechend sekretieren die aktivierten APC Zytokine, welche die Aktivierung der naiven Zellen in der Prägungsphase unterstützen.
3) Prägungsphase: Expression von MHC-Peptid-Komplex und von costimulatorischen Molekülen durch die APC
   Aktivierte APC sekretieren Zytokine, exprimieren costimulatorische Moleküle und den MHC-Peptid-Komplex, entweder de novo und/oder in höheren Konzentrationen. Naive PBMC werden jetzt zu den aktivierten PBMC in gleichen Teilen zugefügt. Aktivierte APC exprimieren zum Beispiel die costimulatorischen B7-Moleküle CD80/CD86, Adhäsionsmoleküle und weitere costimulatorische Moleküle zusammen mit dem MHC-Peptid-Komplex, was zu einer vollständigen Aktivierung der T-Zellen über den αβ-TCR in Effektor- und Gedächtnis-T-Zellen führt. Einige Monozyten aus den frisch zugefügten naiven PBMC differenzieren durch die produzierten Zytokine und/oder durch den Kontakt mit aktivierten T-Zellen wie in 1) oder 2) zu dendritischen Zellen. Es ist vorstellbar, daß dendritische Zellen nicht nur das Gedächtnis der T-Zellen induzieren, sondern auch das Gedächtnis der T-Zellen in der in vivo-Situation durch Präsentation der MHC-Peptid-Komplexe auffrischen.

Der Vorteil der erfindungsgemäßen Verwendung der Kaskaden-Gedächtnisprägung bei der Bereitstellung eines Mittels zur Behandlung von Krebserkrankungen gegenüber allen bisher bekannten Aktivierungsmethoden liegt in der Spezifität und der Geschwindigkeit der Gedächtnis-Induktion in den Lymphozyten. Die Kaskaden-Gedächtnisprägung erfordert nur 24 h bis zum Erhalt der vollständigen zytotoxischen Kapazität der T-Zellen im PBMC-Verband, ohne daß es erforderlich ist, daß die Tumorpeptide/Antigene bekannt sind.

Bei der Erklärung der deutlich erhöhten Wirksamkeit von CAPRI-Zellen im Vergleich zu nur CD3-aktivierten T-Zellen im PBMC-Verband wird u.a. davon ausgegangen, daß, abhängig von der Aktivierungsmethode, eine unterschiedliche Gewichtung der verschiedenen Immunzellen-Subpopulationen eintritt. So können mit Hilfe einer FACS-Analyse (Fluoreszenz-aktivierter Zell-Sorter) quantitative Unterschiede im Auftreten der CD4⁺ T-Zellen (Helferzellen) und der CD8⁺ T-Zellen (zytotoxische Zellen) wie auch der CD45RO⁺ T-Zellen (Gedächtnis-T-Zellen) festgestellt werden. CD3-Aktivierung der PBMC führt zu etwa 25 % CD4⁺ T-Zellen und 58 % CD8⁺ T-Zellen; das Umgekehrte wurde bei der Gedächtnisprägung beobachtet, die zu 54 % CD4⁺ T-Zellen und 29 % CD8⁺ T-Zellen führte. Am bedeutsamsten ist dabei der Anstieg der CD45RO⁺ Gedächtnis-Zellen bei der Kaskaden-Gedächtnisprägung. Nur 3 % der unstimulierten (naiven) PBMC zeigen einen CD45RO⁺ Phänotyp, 29 % der CD3-stimulierten Zellen und 49 % der CAPRI-Zellen. Diese Zunahme an Gedächtniszellen bei der Kaskaden-Gedächtnisprägung ist bei der Bekämpfung von Tumorzellen von großer Bedeutung, da Gedächtnis-Effektorzellen keine oder nur eine sehr geringe Costimulierung für ihre zytotoxische Aktivität benötigen. Ein weiteres Charakteristikum von Gedächtnis-Effektorzellen ist die Fähigkeit zur MHC-restringierten Lyse.

So sind CAPRI-Zellen in der Lage, allogene Krebszelllinien, d.h. Krebszelllinien von anderen, fremden Patienten, hochspezifisch resp. MHC-restringiert zu lysieren, unter der Voraussetzung, daß die allogenen Krebszelllinien und die zytotoxischen CAPRI-Zellen ein gemeinsames MHC-Antigen aufweisen.

Wie bereits erläutert, können mithilfe der Kaskaden-Gedächtnisprägung Gedächtnis-Effektorzellen gegen nicht identifizierte Peptide unbekannter Infektionen erhalten werden. Die genaue Funktion von solchen unbekannten oder manchmal auch bekannten chronischen Infektionen bei der Karzinogenese ist Gegenstand intensiver Forschung. Manche Viren sind bekanntermaßen Hauptfaktoren der Karzinogenese, wie z.B. die humanen Papilloma-Viren (HPV) 16/18 bei Gebärmutterhalskarzinomen. Andere Viren stehen unter einem ähnlichen Verdacht, wenn auch noch keine immunogenen Peptide identifiziert werden konnten. Ein Beispiel dafür ist das JC-Virus, das ein wichtiger karzinogener Faktor bei kolorektalen Karzinomen zu sein scheint. Obschon durch Verabreichung von CAPRI-Zellen eine Regression der kolorektalen Karzinome in Patienten erreicht werden konnte, bleibt unklar, ob die JC-viralen Peptide oder andere Peptide der Tumorzellen die eigentlichen Angriffsziele der lytischen Aktivität der CAPRI-Zellen darstellen.

CAPRI-Zellen können in einer Dosismenge von 0,5-30 Millionen Zellen pro Injektion eingesetzt werden. Die Menge injizierter Zellen kann in Abhängigkeit vom Lebensalter, Körpergewicht und/oder von möglichen Nebenerkrankungen des Patienten angepaßt werden. Es ist möglich, die injizierte Zellmenge mit zunehmender Behandlungsdauer zu erhöhen oder ggf. zu senken. In der Regel liegt die Zellmenge pro Injektion an erwachsene Patienten bei ungefähr 1-20 Millionen Zellen pro Injektion.

Die Injektionen können in unterschiedlichen Zeitabständen, wie ein bis mehrmals wöchentlich, alle paar Wochen oder mit noch längeren Zeitabständen, verabreicht werden.

Die Injektionen von CAPRI-Zellen werden intradermal, intravenös und/oder intramuskulär verabreicht. Falls die Tumorgröße nicht einen Durchmesser von 0,5 cm übersteigt, können die CAPRI-Zellen statt um den Tumor auch in den Tumor verabreicht werden. Bevorzugt erfolgt die Verabreichung über eine kombinierte intradermale und intravenöse Injektion.

Gemäß einer bevorzugten Ausführungform werden den Patienten zusätzlich zu den CAPRI-Zellen CD3-aktivierte Zellen in einer Dosis im Bereich von 1-15 Millionen Zellen verabreicht. Die CD3-aktivierten Zellen können entweder in eine andere Körperstelle als die CAPRI-Zellen oder in die Nähe der intradermalen CAPRI-Infiltrationsstelle verabreicht werden. Die Verabreichung erfolgt bevorzugt über eine intradermale und/oder intramuskuläre Injektion.

Die Behandlung von Krebserkankungen durch die erfindungsgemäße Verwendung von CAPRI-Zellen kann auch zusätzlich zu andersartiger Therapie erfolgen. So können CAPRI-Zellen, gegebenenfalls in Kombination mit CD3-aktivierten Zellen, zur Bereitstellung eines Mittels zur Behandlung von Krebserkrankungen zusätzlich zu üblichen Medikamenten, die im Rahmen einer Chemotherapie verabreicht werden, eingesetzt werden. Des weiteren können diese Zellen zusätzlich zu einer Strahlentherapie verabreicht werden.

### BEISPIELE

### 1. Methoden zur Herstellung von CAPRI-Zellen

### 1) Beginn der CD3-Aktivierungsphase (2-4 h):

10-20 Millionen PBMC (über einen Ficoll-Hypaque-Gradienten aufgetrennt) werden in einem Volumen von 10-12 ml Kulturmedium (wie z.B. RPMI1640) suspendiert, mit 10 % Hyclone-fötalem Kälberserum ergänzt, und auf immobilisierte anti-CD3-monoklonale Antikörper gelagert. Das Hyclone kann jederzeit durch autologes Serum ersetzt werden.

### 2) IL2-Unterstützungsphase (2-3 h):

Nach 2-4 h CD3-Aktivierung werden 20 Einheiten IL-2/ml zugegeben, zur Unterstützung der Aktivierung und zur Verhinderung von Apoptose.

### 3) Prägungsphase (18-24 h):

Nach 2-3 h IL2-Unterstützung sind die APC ausreichend für eine Prägung von naiven PBMC aktiviert: 10-20 Millionen naive PBMC in einem ergänzten Kulturmedium werden zu den stimulierten PBMC zugegeben.

### 4) Expansionsphase (72 h, wahlweise):

Die geprägten PBMC (jetzt CAPRI-Zellen) werden gezählt und in einem ergänzten Medium resuspendiert mit 20 Einheiten IL-2/ml bei einer Konzentration von ca. 0,2-0,4 Millionen Zellen/ml.

### 5) Ernten von CAPRI-Zellen:

Nach 72 h Expansion werden die Zellen geerntet und gefroren in Aliquots von 2-30 Millionen/Ampulle aufbewahrt oder unmittelbar im Rahmen einer Behandlung eingesetzt.

Die Verwendung von allogenen Zellen in den verschiedenen Schritten der Kaskaden-Gedächtnisprägung wie auch andere Modifizierungen können ohne eine Veränderung des Grundverfahrens durchgeführt werden.

Die Herstellung von CAPRI-Zellen und von CD3-aktivierten Zellen wird in der WO99/50393 des Anmelders ausführlich beschrieben.

### 2. Modalitäten der therapeutischen Verabreichung von CAPRI-Zellen

Die CAPRI-Zellen werden in einem geringen Volumen (1 ml) PBS (phosphatgepufferte Salzlösung) resuspendiert und vorzugsweise intradermal und intravenös injiziert. Sie können auch intramuskulär injiziert werden oder um den Tumor, außerdem in den Tumor infiltriert werden, falls die Tumorgröße nicht einen Durchmesser von 0,5 cm übersteigt. Abhängig von der Indikation wird mit Aliquots von ca. 0,5 Millionen CAPRI-Zellen begonnen, pro Injektion sollten aber nicht mehr als 30 Millionen Zellen verabreicht werden.
Werden zusätzlich CD3-aktivierte Zellen verabreicht, so erfolgt die Injektion in einer Dosis von 1-20 Millionen Zellen. Die Verabreichung erfolgt über eine intradermale und/oder intramuskuläre Injektion.

### 3. In vitro-Studien der CAPRI-Zellen Aktivität

A) Solide Krebszelllinien wurden innerhalb von 24 h durch CAPRI-Zellen lysiert. Es zeigte sich, daß CAPRI-Zellen nach Zerstörung einer Krebszelllinie erneut eingesetzt werden können. CAPRI-Zellen konnten bis zu 7 x auf eine weitere Krebszelllinie aufgetragen werden und diese mit derselben Effizienz zerstören. Dabei mußten keine Zytokine zur Unterstützung der CAPRI-Zellen eingesetzt werden. Die getesteten Krebszelllinien stammten von einem Melanom (eine Linie), Brustkarzinom (9 Linien), Dickdarmkarzinom (3 Linien), Glioblastoma multiforme (2 Linien) und Bowenoides Papillom (1 Linie). Insbesondere beim Bowenoid-Tumor zeigte sich die Überlegenheit von CAPRI-Zellen (d.h. naive PBMC geprägt mit aktivierten PBMC des Patienten) im Vergleich mit PBMC-Zellen, die in Gegenwart einer Bowenoid-Tumorzelllinie aktiviert und geprägt worden waren. Erstere waren zur Lyse befähigt, letztere hingegen nicht. Die Zusammensetzung dieser Bowenoid-Tumorzelllinie ist dabei von besonderem Interesse, da diese Tumorzelllinie aus nur etwa 3 % Tumorzellen bestand, die restlichen 97 % waren Fibroblasten. Die Fähigkeit der CAPRI-Zellen, die Krebszellen "hinter" den Fibroblasten zu entdecken, ist vor allem im Zusammenhang mit metastasischen Läsionen, die des öfteren von Fibroblastenschichten umgeben sind, von größter Bedeutung.
   Im Gegensatz zu den CAPRI-Zellen waren die nur CD3-aktivierten Zellen nicht in der Lage, die oben aufgezählten soliden Krebszelllinien zu zerstören. Es muß darauf hingewiesen werden, daß bei frischen Einzelzell-Suspensionen von Tumorbiopsien oder Krebszelllinien schon wiederholt gezeigt werden konnte, daß diese für eine Lyse durch CD3-aktivierte Zellen anfällig sind. Bei diesen Tumorzellen aber handelte es sich um Einzelzell-Suspensionen, die durch enzymatische Behandlung vorgeschädigt waren und die nicht die Möglichkeit hatten, sich zu regenerieren und eine solide Tumorlinie aufzubauen.
B) Bei Assoziationsstudien mit neun Brustkarzinomzelllinien, die aus frischen Tumorbiopsien stammten, zeigte sich eine MHC-Klasse II restringierte Lyse. So lysierten CAPRI-Zellen von einem Donor mit HLA Klasse II Allel DQB1*0201 die autologe Krebszelllinie (auch mit HLA-DQB1*0201) und weitere, allogene Brustkrebszelllinien, die ebenfalls für DQB1*0201 positiv waren. Das gleiche trat bei CAPRI-Zellen auf, die HLA-DQB1*0603 restringiert waren. Nur die HLA-DQB1*0603 positiven autologen Krebszelllinien und weitere Krebszelllinien vom Typ HLA-DQB1*0603 wurden lysiert. Wenn auch die HLA-Klasse II restringierte Lyse bei Brustkarzinomen dominant war, so wurde doch in einem Fall eine HLA-Klasse I restringierte Lyse beobachtet. Antikörper gegen die präsentierenden HLA-Klasse-II-Moleküle blockierten die Krebszellenlyse vollständig, während Antikörper gegen die HLA-Klasse-I-Moleküle die Krebszellenlyse nur schwach verminderten. Die Entfernung von CD4⁺ oder CD8⁺ Immunzellen nach der Kaskaden-Gedächtnisprägung über eine "magnetic bead separation" führte jedoch zum vollständigen Verlust der lytischen Aktivität der zurückgebliebenen CAPRI-Zellen. Im Gegensatz dazu hatte die Entfernung von CD56⁺ oder CD57⁺ Naturalkillerzellen praktisch keinen Einfluß auf die lytische Aktivität. Dies weist darauf hin, daß CAPRI-Zellen auch HLA-Klasse I restringierte Lyse, vorwiegend jedoch HLA-Klasse II restringierte Lyse ausführen. Naturalkillerzellen, die für CD4 oder CD8 positiv sind, aber negativ für CD56 oder CD57, tragen möglicherweise - in geringem Maße - zur lytischen Aktivität der CAPRI-Zellen bei.

### 4. In vivo Studien

A) Die in vivo Wirksamkeit der CAPRI-Zellen konnte anhand einer Untersuchung von zugänglichen Tumoren festgestellt werden. Die folgenden Tumore wurden behandelt und untersucht: ein Melanom, Hautmetastasen von zwei Patientinnen mit Brustkrebs, Hautmetastasen von einer Patientin mit Eierstockkrebs und ein Bowenoides Papillom. Die Applizierungsmethode war eine direkte Injektion in und um den Tumor, falls der Tumor nicht größer als 0,5 cm im Durchmesser war. In größeren Tumoren, z.B. im Fall einer 5 cm x 8 cm Hautmetastase der Patientin mit Eierstockkrebs, wurde die Injektion am Rande des Tumors gesetzt, d.h. im Expansionsbereich. Alle so behandelten Tumore zeigten eine rasche Regression nach 6-8 Wochen mit wöchentlicher oder zweimal wöchentlicher Injektion. Dieses Resultat ist vor allem von Interesse im Fall der Patientin mit dem Bowenoiden Papillom, da diese Patientin fünf mal innerhalb eines Jahres in der dermatologischen Klinik der Universität München laserchirurgisch und durch lokale Applikation von Cremes behandelt worden war. Trotzdem tauchten vor der Injektion von CAPRI-Zellen die Tumorzellen immer wieder im Vulva- und Analbereich auf. Die Patientin ist nun seit zwei Jahren tumorfrei.
B) Im Rahmen der Studie zur in vivo Wirksamkeit von CAPRI-Zellen wurde eine Brustkrebs-Patientin behandelt, die fünf sowohl durch Biopsien wie auch durch Ultraschall und CT nachgewiesene Lebermetastasen aufwies. Dieser Patientin, die während der Periode der CAPRI-Zellenbehandlung keine weitere Behandlung erfuhr, wurden zwei Injektionen pro Woche mit mindestens 20 aber nicht mehr als 30 Millionen CAPRI-Zellen intravenös verabreicht. Erste eindeutige Anzeichen einer Regression des Tumors konnten nach 8 Wochen durch Ultraschall-Untersuchungen festgestellt werden. Die Behandlung mit CAPRI-Zellen induzierte bei 4 von 4 Patienten mit einem metastatischen Dickdarmkarzinom eine signifikante Regression des Tumors. Zwei dieser 4 Patienten erhielten keine Chemotherapie mehr aufgrund der starken Nebenwirkungen und/oder mangelnder Wirksamkeit.
C) Bei den Patienten unter A) und B) wurde entweder keine weitere Medikation verabreicht oder die vorherige Medikation wurde nicht verändert. Zusätzlich zu den CAPRI-Zellen wurden auch CD3-aktivierte Zellen eingesetzt.
D) Die oben beschriebenen Wirkungen der CAPRI-Zellen wurden in autologen Systemen erreicht. Es können jedoch auch allogene (fremde) Zellen in verschiedenen Stadien der Kaskaden-Gedächtnisprägung eingesetzt werden. Dies ist erst bei wenigen Patienten durchgeführt worden, die den Großteil ihrer Immunzellen während der Chemotherapie verloren hatten. Allogene Zellen können manchmal die lytische Aktivität der CAPRI-Zellen intensivieren. Es ist z.B. möglich, für den ersten Schritt der Aktivierung (siehe Methoden zur Herstellung von CAPRI-Zellen, Schritte 1 und 2) die Zellen des Patienten einzusetzen, und dann allogene naive PBMC für eine Prägung mit den autologen aktivierten PBMC des Patienten (Allo-CAPRI) zu verwenden. In einer Familie mit einem Patienten mit einem kolorektalem Karzinom, dessen Vater keine Krebserkrankung entwickelte, bei dem aber mehrfach Polypen im Kolon auftraten, was bekanntlich als Krebsfrühstadium gewertet werden kann, konnten die APC des Vaters (Allo-APC) zur Prägung der naiven Immunzellen des Patienten verwendet werden. In diesem Fall waren die väterlichen APC in der Lage, die naiven PBMC des Patienten zu prägen und so zur Bildung von Gedächtnis-Effektorzellen zu führen, was anhand der Krebszelllinie des Patienten getestet werden konnte. In dieser speziellen familiären Situation konnten auch Allo-allo-CAPRI-Zellen induziert werden, d.h. die aktivierten APC des Vaters konnten die naiven PBMC des Vaters prägen und die so erhaltenen Zellen waren fähig, die Krebszelllinie des Patienten (des Sohnes) zu lysieren. Es muß darauf hingewiesen werden, daß diese väterlichen CAPRI-Zellen, die als Allo-allo-CAPRI-Zellen auf den Krebszelllinien des Patienten verwendet wurden, keinerlei Kontakt mit den Zellen des Patienten hatten, weder mit den APC noch mit den Krebszellen des Patienten, und auch nicht mit aus solchen Krebszellen eluierten Peptiden.

## Patentansprüche

1. Verwendung von mononukleären Zellen des peripheren Blutes (PBMC), umfassend über eine Kaskaden-Gedächtnisprägung stimulierte mononukleäre Zellen des peripheren Blutes mit der Fähigkeit zur HLA-restringierten Lyse (CAPRI-Zellen), erhältlich durch das folgende Stimulierungsverfahren:
- PBMC werden mittels CD3-Antikörper oder mittels immobilisierter CD3-Antikörper primär stimuliert,
- den primär stimulierten PBMC werden naive PBMC zugesetzt und zur Stimulierung der naiven PBMC inkubiert,
wobei die Primärstimulierung der PBMC und/oder die Stimulierung der naiven PBMC in Gegenwart von Interleukin-2 und/oder Interleukin-4 und/oder Interferon γ vorgenommen wird,
zur Bereitstellung eines Mittels zur Behandlung von Krebserkrankungen.

2. Verwendung nach Anspruch 1, **dadurch gekennzeichnet, dass** autologe oder allogene CAPRI-Zellen verwendet werden.

3. Verwendung nach Anspruch 1 oder 2, **dadurch gekennzeichnet, dass** das Verhältnis von primär stimulierten PBMC zu naiven PBMC zwischen 1:10 und 10:1 liegt.

4. Verwendung nach Anspruch 3, **dadurch gekennzeichnet, dass** das Verhältnis von primär stimulierten PMBC zu naiven PBMC bei 1:1 1 liegt.

5. Verwendung nach einem der Ansprüche 1-4, **dadurch gekennzeichnet, dass** die CAPRI-Zellen mittels einer Injektion intradermal, intravenös und/oder intramuskulär und/oder in oder um den Tumor verabreicht werden.

6. Verwendung nach Anspruch 5, **dadurch gekennzeichnet, dass** die CAPRI-Zellen mittels einer Injektion intradermal und intravenös verabreicht werden.

7. Verwendung nach Anspruch 5 oder 6, **dadurch gekennzeichnet, dass** die CAPRI-Zellen in einer Dosis von 0,5-30 Millionen Zellen pro Injektion verabreicht werden.

8. Verwendung nach Anspruch 7, **dadurch gekennzeichnet, dass** die CAPRI-Zellen in einer Dosis von 1-20 Millionen Zellen pro Injektion verabreicht werden.

9. Verwendung nach einem der Ansprüche 1-6, **dadurch gekennzeichnet, dass** die CAPRI-Zellen zusammen mit CD3-stimulierten Lymphozyten verabreicht werden.

10. Verwendung nach Anspruch 9, **dadurch gekennzeichnet, dass** die CD3-stimulierten Lymphozyten in einer Dosis von 1-20 Millionen Zellen pro Injektion verabreicht werden.

11. Verwendung nach Anspruch 9 oder 10, **dadurch gekennzeichnet, dass** die CD3-stimulierten Lymphozyten mittels einer Injektion intradermal und/oder intramuskulär verabreicht werden.

12. Verwendung nach einem der Ansprüche 9-11, **dadurch gekennzeichnet, dass** die CD3-stimulierten Lymphozyten in eine andere Körperstelle des Patienten als die CAPRI-Zellen verabreicht werden.

13. Verwendung nach einem der Ansprüche 1-12, **dadurch gekennzeichnet, dass** sie zusammen mit einer auf den Erkrankungstyp abgestimmten medikamentösen oder strahlentherapeutischen Behandlung erfolgt.

## Claims

1. Use of peripheral-blood mononuclear cells (PBMC), comprising PBMC that are stimulated via cascade priming and are capable of HLA-restricted lysis (CAPRI cells), obtainable by the following stimulation process:
- PBMC are subjected to primary stimulation by means of CD3 antibodies or by means of immobilised CD3 antibodies,
- naïve PMBC are added to the PBMC that have been subjected to primary stimulation and are incubated for stimulating the naive PBMC,
the primary stimulation of the PBMC and/or the stimulation of the naïve PBMC being undertaken in the presence of interleukin-2 and/or interleukin-4 and/or interferon-γ.
for the purpose of providing an agent treating cancerous diseases.

2. Use according to Claim 1, **characterised in that** use is made of autologous or allogenic CAPRI cells.

3. Use according to Claim 1 or 2, **characterised in that** the ratio of PBMC subjected to primary stimulation to naïve PBMC is between 1:10 and 10:1.

4. Use according to Claim 3, **characterised in that** the ratio of PBMC subjected to primary stimulation to naïve PBMC is 1:1.

5. Use according to one of Claims 1-4, **characterised in that** the CAPRI cells are administered by means of an injection intradermally, intravenously and/or intramuscularly and/or in or around the tumour.

6. Use according to Claim 5, **characterised in that** the CAPRI cells are administered by means of an injection intradermally and intravenously.

7. Use according to Claim 5 or 6, **characterised in that** the CAPRI cells are administered in a dosage of 0.5-30 million cells per injection.

8. Use according to Claim 7, **characterised in that** the CAPRI cells are administered in a dosage of 1-20 million cells per injection.

9. Use according to one of Claims 1-6, **characterised in that** the CAPRI cells are administered together with CD3-stimulated lymphocytes.

10. Use according to Claim 9, **characterised in that** the CD3-stimulated lymphocytes are administered in a dosage of 1-20 million cells per injection.

11. Use according to Claim 9 or 10, **characterised in that** the CD3-stimulated lymphocytes are administered by means of an injection intradermally and/or intramuscularly.

12. Use according to one of Claims 9-11, **characterised in that** the CD3-stimulated lymphocytes are administered into a different place in the body of the patient than the CAPRI cells.

13. Use according to one of Claims 1-12, **characterised in that** it is undertaken together with a medicinal or radiotherapeutic treatment that is matched to the type of disease.

## Revendications

1. Utilisation de cellules mononucléaires du sang périphérique (PBMC), comprenant des cellules mononucléaires du sang périphérique stimulées par un processus d'impression en mémoire en cascade qui sont capables de lyse HLA restreinte (cellules CAPRI) et s'obtiennent à l'aide du procédé de stimulation suivant :
- stimulation primaire des PBMC par des anticorps CD3 ou par des anticorps CD3 immobilisés,
- incubation des PBMC stimulées avec lesdits anticorps après addition de PBMC naïves pour permettre la stimulation des PBMC naïves,
la stimulation primaire des PBMC et/ou la stimulation des PBMC naïves s'effectuant en présence d'interleukine 2 et/ou d'interleukine 4 et/ou d'interféron y,
pour préparer un agent permettant de traiter des affections cancéreuses.

2. Utilisation selon la revendication 1, **caractérisée en ce qu'**il est utilisé des cellules CAPRI autologues ou allogéniques.

3. Utilisation selon la revendication 1 ou 2, **caractérisée en ce que** le rapport entre les PBMC premièrement stimulées et les PBMC naïves varie entre 1/10 et 10/1.

4. Utilisation selon la revendication 3, **caractérisée en ce que** le rapport entre les PBMC premièrement stimulées et les PBMC naïves est de 1/1.

5. Utilisation selon l'une des revendications 1 à 4, **caractérisée en ce que** les cellules CAPRI sont administrées par injection intradermique, intraveineuse et/ou intramusculaire et/ou dans ou autour de la tumeur.

6. Utilisation selon la revendication 5, **caractérisée en ce que** les cellules CAPRI sont administrées par injection intradermique et intraveineuse.

7. Utilisation selon la revendication 5 ou 6, **caractérisée en ce que** la dose des cellules CAPRI administrée est de 0,5 à 30 millions de cellules par injection.

8. Utilisation selon la revendication 7, **caractérisée en ce que** la dose des cellules CAPRI administrée est de 1 à 20 millions de cellules par injection.

9. Utilisation selon l'une des revendications 1 à 6, **caractérisée en ce que** des lymphocytes stimulés par CD3 sont administrés en même temps que les cellules CAPRI.

10. Utilisation selon la revendication 9, **caractérisée en ce que** la dose des lymphocytes stimulés par CD3 administrée est de 1 à 20 millions de cellules par injection.

11. Utilisation selon la revendication 9 ou 10, **caractérisée en ce que** les lymphocytes stimulés par CD3 sont administrés par injection intradermique et/ou intramusculaire.

12. Utilisation selon l'une des revendications 9 à 11, **caractérisée en ce que** les lymphocytes stimulés par CD3 sont administrés à un autre endroit du corps du patient que les cellules CAPRI.

13. Utilisation selon l'une des revendications 1 à 12, **caractérisée en ce qu'**elle est effectuée conjointement avec un traitement radiothérapeutique ou médicamenteux adapté au type d'affection à traiter.
